# EUROPEAN PATENT APPLICATION

(11) **EP 1 237 100 A1**
(43) Date of publication of application: **04.09.2002**
(21) Application number: 00951978.6
(22) Date of filing: 14.08.2000
(51) Int. Cl.: G06F 17/60

(54) **COSMETIC ORDER-PRODUCTION SYSTEM IN RESPONSE TO LIKING OF CUSTOMER AND SHOP WITH SMALL-SCALE COSMETIC FACTORY**

(30) Priority: 19.08.1999 JP 23227399; 13.12.1999 JP 35355599
(71) Applicant: Shu Uemura Cosmetics Inc., Tokyo 107-0062 (JP)
(72) Inventor: Uemura, Shu Shu Uemura Cosmetics Inc., Minato-ku, Tokyo 107-0062 (JP)
(74) Representative: Kédinger, Jean-Paul
(86) International application number: JP0005434
(87) International publication number: WO01015029

(57) **Abstract**

The present invention is a system for customizing cosmetics to customer needs for inquiring of a customer about needs on cosmetics by a cosmetic sales person in a shop having an annexed small-scale cosmetic plant and preparing cosmetics, the small-scale cosmetic plant including: means for blending materials based on an optimum formula for the needs to prepare a small amount of cosmetic bulk; means for molding the blended cosmetic bulk if necessary; and means for setting the cosmetic bulk or cosmetic mold into a container or a case. According to the present invention, it is possible to resolve drawbacks associated with conventional sales styles of cosmetics and to sell cosmetics adequate for customer needs promptly without an inventory stock of items in the shop.

## Description

### Technical Field

The invention relates to a system for customizing cosmetics for manufacturing and selling cosmetics adequate for customer needs, and a shop having an annexed small-scale cosmetic plant for that purpose.

### Technical Background

In conventional sales styles of cosmetics, it has been common to display a number of samples of merchandise in a shop so that customers select and purchase items based on the samples of merchandise or on the basis of counseling from an adviser over the counter.

To satisfy customer needs in such sales styles, however, various kinds of items had to be stocked in the shop with tiresome operation and maintenance. Furthermore, customers could only select cosmetics from the stock in the shop, and had no choice but to forgo purchasing if no cosmetics congenial to their tastes were found in the shop stock. This has lead up to loss of sales opportunities.

Among the styles of selling cosmetics according to customer needs is one to inquire of visiting customers about skin properties and needs, followed by manufacture in a manufacturing plant and delivery to the customers later. Such a sales style had a drawback, however, that it would take quite a few days between the order from customers and the delivery to the customers.

### Disclosure of the Invention

A problem to be solved by the present invention is to provide: a system for customizing cosmetics which can resolve the drawbacks associated with the conventional sales styles of cosmetics and sell cosmetics adequate for customer needs speedily without keeping an inventory stock of items in a shop; a made-to-order system for meeting repeat orders; and a shop for that purpose.

Another problem to be solved by the present invention is to provide a system for customizing cosmetics capable of satisfying the demand of customers for having cosmetics for their own use immediately on the spot.

The present invention to solve the foregoing problems relates to a system for customizing cosmetics to customer needs for inquiring of a customer about needs on cosmetics by a cosmetic sales person in a shop having an annexed small-scale cosmetic plant and preparing cosmetics, the small-scale cosmetic plant including: means for blending materials based on an optimum formula for the needs to prepare a small amount of cosmetic bulk; means for molding the blended cosmetic bulk to be arranged if necessary; and means for setting the cosmetic bulk or cosmetic mold into a container or a case.

Another invention to solve the foregoing problems relates to a made-to-order system of cosmetics for meeting a repeat order from a customer, comprising: means for blending materials based on a formula stored in storage means in the small-scale cosmetic plant to prepare a small amount of cosmetic bulk in response to a repeat order from a customer; means for molding the blended cosmetic bulk to be arranged if necessary; and means for setting the cosmetic bulk or cosmetic mold into a container or a case.

Still another invention to solve the forgoing problems relates to a shop-annexed plant for preparing cosmetics adequate for customer needs, comprising: a shop for inquiring of a customer about cosmetics; and a small-scale cosmetic plant arranged next to the shop, the small-scale cosmetic plant including input means for inputting an optimum formula for the needs, storage means for storing the formula input, output means for outputting the formula stored, means for blending materials based on the formula output to prepare a small amount of cosmetic bulk, means for molding the cosmetic bulk blended to be arranged if needed, and mean for setting the cosmetic bulk or cosmetic mold into a container or a case.

The applicable cosmetics of the customization system according to the present invention include makeup cosmetics such as face powders, powders, foundations, pencils, blushers, eye shadows, lipsticks, and chapsticks, and basic skin cares such as general creams/emulsions, lotions, and colognes. The present invention is suited to makeup cosmetics.

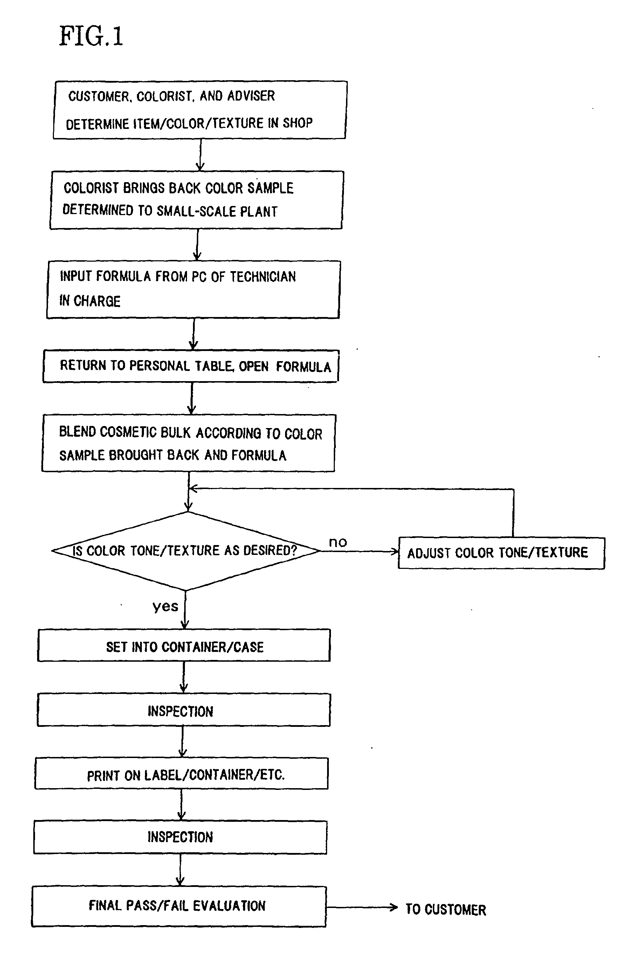

Fig. 1 is a flowchart showing an embodiment of the system for customizing cosmetics according to the present invention.

Although not shown, an adviser, who is a cosmetic sales person behind the counter, first explains the customization system of the present invention to a customer visiting the shop. Then, the adviser and a colorist, as counselors, inquire about customer needs through counseling, and determine cosmetic items, color, and texture.

The colorist inputs formula data from a personal computer of a manufacturing technician in charge in a small-scale cosmetic plant annexed to the shop. The formula input is stored in a network-connected server or host computer, along with customer information. Since formulae are stored in the server or host computer customer by customer, it is possible to accept repeat orders from the same customers by phone, by fax, and/or over the Internet for reproduction on the basis of the formulae stored.

Alternatively, the customer formulae may be filed and stored on paper media such as cards instead of the computer system.

The manufacturing technician in charge of the cosmetics returns to his/her table in the small-scale cosmetic plant annexed to the shop, and outputs the formula to a display or a printer from the personal computer which is connected to the server or host computer storing the formula.

According to a color sample brought by the colorist and the formula, the chief manufacturing technician blends materials by using blending means. The materials include raw materials, semimanufactured products, and cosmetic bulks.

The raw materials are not limited to particular ones as long as they make cosmetic raw materials. Examples thereof include: hydrocarbons such as squalane, liquid paraffin, a-olefin oligomer, paraffin wax, ceresin, and microcrystalline wax; animal and vegetable oils such as corn oil, avocado oil, persic oil, olive oil, beef tallow, and mink oil; liquid and solid synthetic esters such as isopropyl myristate, cetyl octoate, and cetyl palmitate; natural animal and vegetable wax such as jojoba oil, carnauba wax, candelila wax. Japan wax, and bees wax; surfactants such as glyceryl stearate, sorbitan stearate, polyoxyethylene sorbitan monooleate, polyoxyethylene glyceryl tristearate, polyoxyethylene lauryl ether, decaglyceryl trioleate, monolauric acid sucrose ester, and polyoxyethylene-hardened caster oil: silicone oils and their derivatives such as dimethyl polysiloxane, and methylphenyl polysiloxane; fluorine-type resins such as perfluoropolyether; alcohls such as ethanol, isopropanol, ethyleneglycol, glycerol, 1,3-butyleneglycol, propyleneglycol, and diglycerol; water-soluble polymers such as carboxyvinylpolymer, carrageenan, xanthan gum, sodium carboxymethylcellulose, and sodium hyaluronate; proteins such as collagen, elastin, silk, and lactoferrin; powders such as titanium dioxide, zinc oxide, talc, mica, silicic anhydride, nylon powder, alkyl polyacrylate, alumina, and ferric oxide; and other additives such as UV absorbers, vitamins, anti-inflammatory agents, amino acids and their derivatives, lecithin, colorants, perfumes, and antiseptic agents. These raw materials may be mixed if necessary.

The cosmetic bulks serving as materials refer to homogenous mixtures of raw materials that are obtained through, for example, dissolution, three-roll kneading, pulverization, and mixer blending, and yet to be charged into final containers nor molded for loading. The resultants can be turned into end products when charged into final containers or loaded after molding.

In the small-scale cosmetic plant, a small amount of cosmetic bulk may be prepared from raw materials. For the reduction of time, semimanufactured products, each containing several types of raw materials and coloring matters mixed in advance, may be blended into cosmetics of desired color tone instead of the fabrication from the raw materials. The semimanufactured products in the present invention refer to cosmetic bulks of plain colors, which can make end products by themselves. The plain colors include red, blue, yellow, white, and black. Semimanufactured products in different colors can be blended to prepare cosmetic bulks of any color. For example, a semimanufactured product in red (for lipstick) and a semimanufactured product in blue (for lipstick) can be blended into a cosmetic bulk in purple (for lipstick). The blended cosmetic bulk is molded into a stick form to make a purple lipstick. Modifying the compounding ratio between the red color base and the blue color base allows the preparation of purple in any color tone. In the present invention, the apparatus for blending may be compact in size because customer orders come in small lots such as one to several pieces.

The preparation means of cosmetic bulks depend on the cosmetics. For example, in the case of solid cosmetics such as lipsticks, it is essential only that raw materials or semimanufactured products heat-melted be agitated for blending before poured into a mold, cooled into sticks, and loaded into lipstick cases.

In the case of solid foundations, though depending on the aspect, it is essential only that raw materials or semimanufactured products heat-melted or not heat-melted be agitated for blending, or mulled for blending, before poured into a metal plate or the like and loaded into containers.

For powdered cosmetics such as face powders, raw materials or semimanufactured products have only to be mixed in a mixer or the like, pulverized in some cases, and blended before put in a mold, press-molded, and set in containers. Alternatively, the blended article may be loaded into containers without the molding step.

For liquid cosmetics such as lotions and emulsions, liquid cosmetic bulks are blended and then filled into containers to complete the cosmetics without a molding step.

The apparatuses, tools, and the like to be used for preparing cosmetic bulks, molding cosmetic bulks, or accommodating molded cosmetics into containers may be ones for manufacturing ordinary cosmetics, selected as appropriate depending on such conditions as the types of cosmetics to fabricate and the size of the plant.

The completed cosmetics are inspected, and then necessary information is printed on the cosmetics themselves, labels, containers, paper bags, and so on by printing means. The printing means include a laser marker, an ink-jet printer, and handwriting. Alternatively, labels printed in advance may be pasted on. The information to be printed includes lot number, color name (number), best-before date, customer name, colorist name, and adviser name. After printed, the completed cosmetics are passed through final inspection and handed to the customers.

According to the customization system of the present invention described above, it becomes possible for customers to bring containers they like or they made to the shop so as to have these containers filled with products. Needless to say, it is also possible to sell original containers at the shop to have these containers filled with.

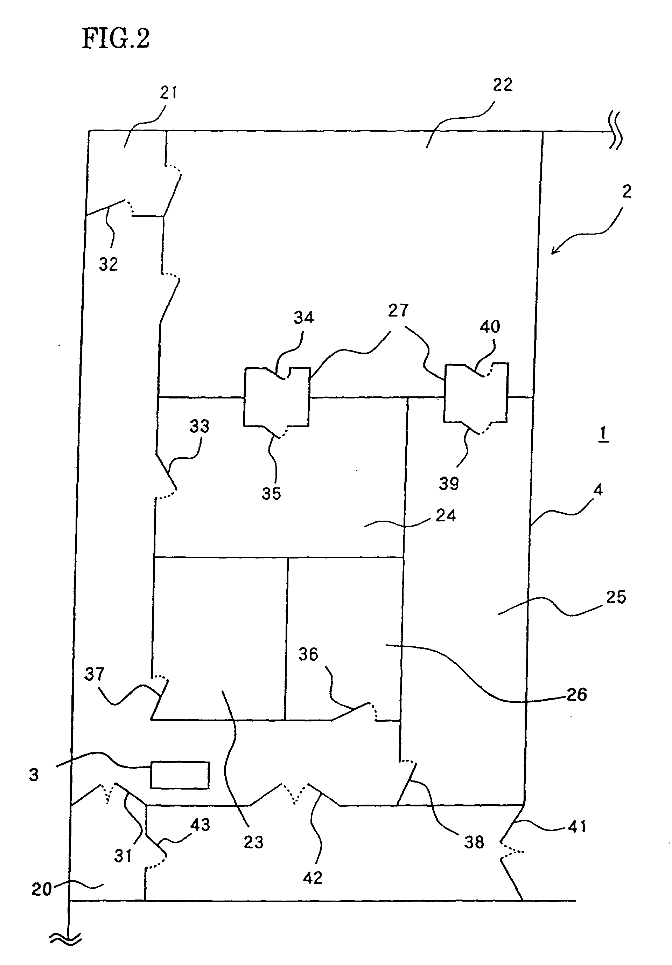

Fig. 2 is a plan view showing an example of the shop having an annexed small-scale cosmetic plant according to the present invention.

The shop having an annexed small-scale cosmetic plant of the present invention comprises the area of a shop 1 and the area of a small-scale cosmetic plant 2 annexed to the shop 1. The shop 1 has a not-shown counseling space in which the adviser and the colorist provide counsel for a customer. In the counseling space of the shop 1, items/colors/textures adequate for customer needs are determined, optimum formulae for the needs are determined, and color samples are created.

In addition, the shop 1 has a display to showcase a variety of products so that the advisor can give consultation in the counseling space as to cosmetics other than the products under blending. Needless to say, the consultation to reflect one's needs to the blended products leads to important sales promotions.

For example, the colorist brings back a formula and a color sample to the small-scaled cosmetic plant. He/she enters the small-scaled cosmetic plant through entrance doors 41 and 43, changes into dust preventive clothing in a dressing room 20, washes his/her hands in a wash basin 3, and goes into a test inspection room 24 through a door 33. In the test inspection room 24, the colorist initially inputs the customer information and the formula to a not-shown computer.

Next, the colorist passes through a door 32 to enter a dressing room 21 to further change into dust preventive clothing and footwear, returns to his/her own table in an adjustment operation room 22, and outputs the formula from a computer which is connected to the formula-input computer via the network. The colorist blends materials such as raw materials and semimanufactured products to prepare a cosmetic bulk according to the color sample brought back to the adjustment operation room 22 and the formula.

In the test inspection room 24, the colorist compares the cosmetic bulk prepared once with the color sample to check for the desired color tone and texture. If the color tone and texture are not as desired, readjustment is performed.

A pre-clean room 27 is arranged between the adjustment operation room 22 and the test inspection room 24, preventing dust move therebetween. Another pre-clean room is arranged between the adjustment operation room and a material storage room 25.

When a window pane is arranged in a partition 4 between the shop 1 and the small-scale cosmetic plant 2, or when the partition 4 is made of glass, the customer can see how their ordered cosmetics are being prepared in the small-scale cosmetic plant 2.

After the adjustment of the color tone and texture, the cosmetic bulk is molded according to the type of the cosmetics, and set in containers or cases. The completed cosmetics are carried into the test inspection room 24 for necessary inspections.

After inspected, the completed products are carried into a packing room 23 so that necessary information is printed on the cosmetics themselves, containers, paper bags, and so on.

Finally, an inspection is performed in the test inspection room 24, and the completed cosmetics are handed to the customer.

Incidentally, the raw materials, semimanufactured products, and cosmetic bulks serving as the materials can be stored in the material storage room 25 through doors 42 and 38.

### Brief Explanation of the Drawings

Fig. 1 is a flowchart showing an example of the system for customizing cosmetics according to the present invention; and
Fig. 2 is a plan view showing an example of the shop having an annexed small-scale cosmetic plant according to the present invention.

The reference numerals in the drawings represent the following:
1: shop; 2: small-scale cosmetic plant; 3: wash basin; 4: partition; 21: dressing room; 22: adjustment operation room; 23: packing room; 24: test inspection room; 25: material storage room; 26: materials room; 27: pre-clean room; and 31, 32, 33, 34, 35, 36, 37, 38, 39, 40, 41, 42, and 43: door.

### Best Mode for Carrying Out the Invention

### Embodiments

Hereinafter, the present invention will be described in details in conjunction with embodiments, whereas the present invention will not be limited to these embodiments.

### Embodiment 1

**Table 1**

| Raw material | |
|---|---|
| Compounded amount (parts by weight) | |
| (1) | sericite |
| (2) | talc |
| (3) | light calcium carbonate |
| (4) | titanium oxide |
| (5) | iron oxide yellow |
| (6) | iron oxide red |
| Total amount | |

Table 1 shows examples of formulae for face powders. In the small-scale plant annexed to the shop, face powders were prepared to the compositions shown in Table 1.

In the absence of oil solution as in Table 1, it is possible to perform measuring, mixing, pulverization, sifting, and filling by using small-scale equipment in the small-scale plant annexed to the shop.

### Embodiment 2

Bulks of semimanufactured products 1-5 of plain-color lipsticks as shown in Table 2 were prepared beforehand in another plant without molding.

**Table 2**

| Raw material | |
|---|---|
| Compounded amount (parts by weight) | |
| Semimanufactured product | |
| (1) | ceresin |
| (2) | carnauba wax |
| (3) | methylphenyl polysiloxane |
| (4) | castor oil |
| (5) | squalane |
| (6) | natural vitamin E |
| (7) | titanium oxide |
| (8) | barium sulfate |
| (9) | red No. 226 |
| (10) | yellow No. 5 (aluminum lake) |
| (11) | perfume |
| Total amount | |

(Rest of Page is Blank)

**Table 3**

| Raw materials | |
|---|---|
| Compounded amount (parts by weight) | |
| Embodiment | |
| Comparative example | |
| (1)-(5) | Semimanufactured product |
| (6) | ceresin |
| (7) | carnauba wax |
| (8) | methylphenyl polysiloxane |
| (9) | castor oil |
| (10) | squalane |
| (11) | natural vitamin E |
| (12) | titanium oxide |
| (13) | barium sulfate |
| (14) | red No. 226 |
| (15) | yellow No. 5 (aluminum lake) |
| (11) | perfume |
| Total amount | |

In the parentheses, they are shown as compounding ratios in terms of each of the raw materials in the embodiment.

Then, in the small-scale plant annexed to the shop, the products of the embodiment were prepared to the compositions shown in Table 3.

Meanwhile, in another cosmetic manufacturing plant, raw materials were manufactured to the compositions of the comparative example by using advanced facilities for measuring, dissolution, kneading, and degassing. The cosmetics of the comparative example took time for the manufacturing process, and required sophisticated inspections including material inspection and bulk inspection.

In contrast, according to the embodiment, the components had only to be measured, dissolved, and mixed for completion.

Besides, in the embodiment, the mixtures could be obtained in amounts from as small as several grams. It was possible to fabricate merely one to several pieces according to customer needs.

On the contrary, in the comparative example, the products came in large amounts. The result is that when the customer needs are in smaller amounts, most of the manufactured bulks are wasted.

### Embodiment 3

Semimanufactured products 1, 3, 6 as shown in Table 4 were prepared.

**Table 4**

| Raw materials | |
|---|---|
| Compounded amount (parts by weight) | |
| Semimanufactured product | |
| (1) | ceresin |
| (2) | carnauba wax |
| (3) | methylphenyl polysiloxane |
| (4) | castor oil |
| (5) | squalane |
| (6) | natural vitamin E |
| (7) | titanium oxide |
| (8) | red No. 226 |
| Total amount | |

In Table 4, the semimanufactured product 1 and the semimanufactured product 3 are the same as those of Table 2. The product 6 contains no power material and have a rather hard feel.

Next, these semimanufactured products were used to prepare cosmetics in the small-scale cosmetic plant, according to the compositions as shown in Table 5.

**Table 5**

| Raw materials |
|---|
| Semimanufactured product |
| mica |
| squalane |
| titanated mica |
| Total amount |

A is a mixture of semimanufactured products given toning alone. B to E could be obtained through simple operations of adding other materials to this A, followed by dissolution, degassing, and molding.

As compared with A, the lipsticks of different feels obtained as above, or B, C, D, and E, respectively offer: a high hardness for stay-put; a powdery, matte finish; a clear, glossy, light finish; and a pearly finish. These embodiments can be easily modified in feel by adjusting the amounts of additives to the semimanufactured products 1.

In the present invention, simply adding necessary semimanufactured products of plain colors will not cause any change in feel or physical property. Furthermore, since the semimanufactured products of plain colors can be used to complete this adjusting operation in a few minutes, customers will not be kept waiting long.

### Industrial Applicability

According to the present invention, cosmetics adequate for customer needs can be fabricated and sold right on the spot without a large stock of products in the shop. This can satisfy the demand of customers for having cosmetics for their own use immediately on the spot. Moreover, the small-scale cosmetic plant annexed to the shop is where semimanufactured products fabricated in another plant are simply mixed and blended; besides, customer orders come in small amounts. Therefore, the apparatuses for blending, molding, and filling in the small-scale cosmetic plant may be small ones, which allows a reduction in the investments in equipment.

When a paned window is arranged in the partition between the annexed small-scale cosmetic plant and the shop, or when the partition is made of glass, blending operations can be demonstrated to customers for improved visual attraction.

In addition, the formulae determined by the expert counseling are stored into the server or the host computer. This facilitates meeting repeat orders from customers by phone, by facsimile, and/or over the Internet.

Furthermore, it is possible for customers to have their favorite containers filled with cosmetics of their own.

## Claims

1. A system for customizing cosmetics to customer needs, comprising a shop and an annexed small-scale cosmetic plant for preparing cosmetics, a cosmetic sales person inquiring of a customer about needs on cosmetics in said shop, said small-scale cosmetic plant including: means for blending materials based on an optimum formula for said needs to prepare a small amount of cosmetic bulk; means for molding said blended cosmetic bulk to be arranged if necessary; and means for setting said cosmetic bulk or cosmetic mold into a container or a case.

2. The system for customizing cosmetics according to claim 1, wherein said materials are cosmetic raw materials, semimanufactured products, or cosmetic bulks.

3. The system for customizing cosmetics according to claim 1 or 2, wherein for said needs of the customer, the cosmetic sales person determines a cosmetic item, color, and texture through counseling with the customer.

4. The system for customizing cosmetics according to any one of claims 1-3, further comprising: input means for inputting said optimum formula for said needs of the customer; storage means for storing said formula input; and output means for outputting said formula stored.

5. The system for customizing cosmetics according to any one of claims 1-4, wherein: said input means, said storage means, and said output means are composed of a computer system; and a plurality of computers are connected through a network.

6. The system for customizing cosmetics according to any one of claims 1-5, wherein said semimanufactured products are cosmetic bulks of plain colors.

7. The system for customizing cosmetics according to any one of claims 1-6, further comprising print means for printing arbitrary information onto cosmetics set in said container or case, said container, or the like.

8. The system for customizing cosmetics according to claim 7, wherein said print means are laser print means.

9. A made-to-order system of cosmetics for meeting a repeat order from a customer, comprising: means for extracting a formula of a customer stored in said storage means according to claim 4 or 5 and blending materials based on said formula to prepare a small amount of cosmetic bulk in response to a repeat order from the customer; means for molding said blended cosmetic bulk to be arranged if necessary; and means for setting said cosmetic bulk or cosmetic mold into a container or a case.

10. The made-to-order system of cosmetics for meeting a repeat order from a customer according to claim 9, wherein said repeat order is given by phone, by facsimile, or over the Internet.

11. A shop having an annexed small-scale cosmetic plant for preparing cosmetics adequate for customer needs, comprising: a shop for a cosmetic sales person to inquire of a customer about cosmetics; and a small-scale cosmetic plant arranged next to said shop, said small-scale cosmetic plant including input means for inputting an optimum formula for the needs, storage means for storing said formula input, output means for outputting said formula stored, means for blending materials based on said formula output to prepare a small amount of cosmetic bulk, means for molding said cosmetic bulk blended to be arranged if needed, and mean for setting said cosmetic bulk or cosmetic mold into a container or a case.
